# EUROPEAN PATENT APPLICATION

(11) **EP 4 821 944 A1**
(43) Date of publication of application: **14.10.2026**
(21) Application number: 24900035.7
(22) Date of filing: 20.12.2024
(51) Int. Cl.: C07K 14/78, C12N 15/12, A61K 8/65, A61Q 19/08, A61Q 7/00, A61Q 5/12, A61P 17/02, A61K 38/39, A61L 27/24, A61L 27/50

(54) **RECOMBINANT TYPE XVII COLLAGEN HAVING TRIPLE HELIX STRUCTURE AND USE THEREOF**

(30) Priority: 05.12.2023 CN 202311658196
(71) Applicant: Bloomature Biotechnology Co., Ltd., Beijing 102629 (CN)
(72) Inventor: ZHANG, Junli, Beijing 102629 (CN); WANG, Ruiyan, Beijing 102629 (CN); TAO, Wenwen, Beijing 102629 (CN); MU, Shicheng, Beijing 102629 (CN); GUO, Haixin, Beijing 102629 (CN); HAN, Yingchao, Beijing 102629 (CN); ZHOU, Jingwen, Beijing 102629 (CN); WANG, Xinglong, Beijing 102629 (CN); ZHAO, Xinyi, Beijing 102629 (CN); YU, Shuyao, Beijing 102629 (CN); LI, Yiheng, Beijing 102629 (CN); SUN, Xin, Beijing 102629 (CN); HAO, Aijun, Beijing 102629 (CN); PENG, Fan, Beijing 102629 (CN); DU, Jingyi, Beijing 102629 (CN); YANG, Shuang, Beijing 102629 (CN)
(74) Representative: Huang, Liwei
(86) International application number: PCT/CN2024/141080
(87) International publication number: WO 2025/119402

(57) **Abstract**

Provided in the present invention is a recombinant type XVII collagen having a triple helix structure. The collagen has an amino acid sequence containing one or more amino acid residue repeating units, wherein each of the repeating units contains a sequence as shown in SEQ ID NO.1 or SEQ ID NO.2, and the repetition number is n, where 1<n≤60. The recombinant type XVII collagen provided in the present invention can be widely used in the fields of food, health care products, cosmetics, medical devices, etc.

## Description

### CROSS-REFERENCE TO RELATED INVENTION

This invention claims priority to Chinese Patent Application No. 202311658196.9, filed with the China National Intellectual Property Administration on December 5, 2023 and entitled "RECOMBINANT TYPE XVII COLLAGEN HAVING TRIPLE HELIX STRUCTURE AND USE THEREOF", the entire contents of which are incorporated herein by reference.

### TECHNICAL FIELD

The present invention relates to the technical field of biological proteins, particularly to a recombinant type XVII collagen having a triple helix structure and use thereof.

### BACKGROUND

There are 28 types of collagen in total, accounting for approximately 30% of the total protein in the human body. Collagen is mainly distributed in the skin, tendons, bones, blood vessels, cornea and other tissues, with a molecular weight ranging from 50 to 340 kDa. Among them, type XVII collagen is predominantly located in regions such as skin and epithelium, and is a transmembrane protein composed of three α chains each of which has a size of 180 kDa. The type XVII collagen can mediate the interactions of stem cells and surrounding cells with a matrix, exhibits functions in regulating skin homeostasis, senescence, wound repair and the like, and has broad application prospects in the fields of cosmetics and medical devices.

In the human skin, collagen primarily provides a structural support. This supporting function mainly depends on the triple helix structure of collagen. At present, a recombinant collagen is mainly produced by using a microbial fermentation method. For example, in patent CN113185604A and patent CN110845603A, a recombinant type XVII collagen is designed and expressed by using yeast cells and Escherichia coli cells, but it does not exhibit the triple helix structure. The synthesis of a collagen having a correct triple helix structure in microbial fermentation by utilizing a synthetic biology technology has a relatively high technical barrier. Therefore, the design and construction of the recombinant type XVII collagen having the triple helix structure is still a technical challenge that needs to be conquered for producing the recombinant collagen in microbial fermentation production.

### SUMMARY

To solve the above problem, the present invention is intended to provide a recombinant type XVII collagen having a triple helix structure, which is simple in preparation, high in expression level and good in efficacy, and is capable of achieving self-assembly.

In one aspect, the present invention provides a recombinant type XVII collagen, wherein the collagen has an amino acid sequence containing one or more amino acid residue repeating units, wherein the repeating unit comprises a sequence as shown in SEQ ID NO. 1, and the repetition number is n, where 1<n≤60.

Optionally, the repetition number n may be in the range of 1<n≤60, 2≤n≤60, 3≤n≤60, 4≤n≤60, 5≤n≤60, 6≤n≤60, 7≤n≤60, 8≤n≤60, 9≤n≤60, 10≤n≤60, 11≤n≤60, 12≤n≤60, 13≤n≤60, 14≤n≤60, 15≤n≤60, 16≤n≤60, 17≤n≤60, 18≤n≤60, 19≤n≤60, 20≤n≤60, 21≤n≤60, 22≤n≤60, 23≤n≤60, 24≤n≤60, 25≤n≤60, 26≤n≤60, 27≤n≤60, 28≤n≤60, 29≤n≤60, 30≤n≤60, 31≤n≤60, 32≤n≤60, 33≤n≤60, 34≤n≤60, 35≤n≤60, 36≤n≤60, 37≤n≤60, 38≤n≤60, 39≤n≤60, 40≤n≤60, 41≤n≤60, 42≤n≤60, 43≤n≤60, 44≤n≤60, 45≤n≤60, 46≤n≤60, 47≤n≤60, 48≤n≤60, 49≤n≤60, 50≤n≤60, 51≤n≤60, 52≤n≤60, 53≤n≤60, 54≤n≤60, 55≤n≤60, 56≤n≤60, 57≤n≤60, 58≤n≤60, 59≤n≤60, 1≤n≤10, 10≤n≤20, 20≤n≤30, 30≤n≤40, 40≤n≤50, 50≤n≤60, 5≤n≤15, 15≤n≤25, 25≤n≤35, 35≤n≤45, 45≤n≤55, 55≤n≤60, 1≤n≤10, 10≤n≤20, 20≤n≤30, 30≤n≤40, 40≤n≤50, 50≤n≤60, 5≤n≤15, 15≤n≤25, 25≤n≤35, 35≤n≤45, 45≤n≤55, 55≤n≤60, 10≤n≤30, 30≤n≤60, 5≤n≤30, 30≤n≤40, 40≤n≤50, 50≤n≤60, 5≤n≤15, 15≤n≤25, 25≤n≤35, 35≤n≤45, 45≤n≤55, 55≤n≤60, 1≤n≤55, 5≤n≤55, 10≤n≤60, 15≤n≤60, 25≤n≤60, 35≤n≤60, 1≤n≤4, 2≤n≤4, 3≤n≤4, 1≤n≤8, 2≤n≤8, 3≤n≤8, 4≤n≤8, 5≤n≤8, 6≤n≤8, 7≤n≤8, 1≤n≤16, 2≤n≤16, 3≤n≤16, 4≤n≤16, 5≤n≤16, 6≤n≤16, 7≤n≤16, 8≤n≤16, 9≤n≤16, 10≤n≤16, 11≤n≤16, 12≤n≤16, 13≤n≤16, 14≤n≤16, 15≤n≤16, 1≤n≤20, 2≤n≤20, 3≤n≤20, 4≤n≤20, 5≤n≤20, 6≤n≤20, 7≤n≤20, 8≤n≤20, 9≤n≤20, 10≤n≤20, 11≤n≤20, 12≤n≤20, 13≤n≤20, 14≤n≤20, 15≤n≤20, 16≤n≤20, 17≤n≤20, 18≤n≤20, 19≤n≤20, 1≤n≤30, 2≤n≤30, 3≤n≤30, 4≤n≤30, 5≤n≤30, 6≤n≤30, 7≤n≤30, 8≤n≤30, 9≤n≤30, 10≤n≤30, 11≤n≤30, 12≤n≤30, 13≤n≤30, 14≤n≤30, 15≤n≤30, 16≤n≤30, 17≤n≤30, 18≤n≤30, 19≤n≤30, 20≤n≤30, 21≤n≤30, 22≤n≤30, 23≤n≤30, 24≤n≤30, 25≤n≤30, 26≤n≤30, 27≤n≤30, 28≤n≤30, 29≤n≤30, 1≤n≤35, 2≤n≤35, 3≤n≤35, 4≤n≤35, 5≤n≤35, 6≤n≤35, 7≤n≤35, 8≤n≤35, 9≤n≤35, 10≤n≤35, 11≤n≤35, 12≤n≤35, 13≤n≤35, 14≤n≤35, 15≤n≤35, 16≤n≤35, 17≤n≤35, 18≤n≤35, 19≤n≤35, 20≤n≤35, 21≤n≤35, 22≤n≤35, 23≤n≤35, 24≤n≤35, 25≤n≤35, 26≤n≤35, 27≤n≤35, 28≤n≤35, 29≤n≤35, 4≤n≤40, 4≤n≤24, 4≤n≤8, 4≤n≤16, 4≤n≤24, 8≤n≤16, 8≤n≤24, 8≤n≤40, 16≤n≤24, 16≤n≤40, 24≤n≤40, 30≤n≤35, 30≤n≤40, 35≤n≤40 and any range therebetween.

In another aspect, the present invention further provides a recombinant type XVII collagen, wherein the collagen has an amino acid sequence containing one or more amino acid residue repeating units, wherein the repeating unit comprises a sequence as shown in SEQ ID NO.2, and the repetition number is n, where 1<n≤60.

Optionally, the repetition number n may be in the range of 1<n≤60, 2≤n≤60, 3≤n≤60, 4≤n≤60, 5≤n≤60, 6≤n≤60, 7≤n≤60, 8≤n≤60, 9≤n≤60, 10≤n≤60, 11≤n≤60, 12≤n≤60, 13≤n≤60, 14≤n≤60, 15≤n≤60, 16≤n≤60, 17≤n≤60, 18≤n≤60, 19≤n≤60, 20≤n≤60, 21≤n≤60, 22≤n≤60, 23≤n≤60, 24≤n≤60, 25≤n≤60, 26≤n≤60, 27≤n≤60, 28≤n≤60, 29≤n≤60, 30≤n≤60, 31≤n≤60, 32≤n≤60, 33≤n≤60, 34≤n≤60, 35≤n≤60, 36≤n≤60, 37≤n≤60, 38≤n≤60, 39≤n≤60, 40≤n≤60, 41≤n≤60, 42≤n≤60, 43≤n≤60, 44≤n≤60, 45≤n≤60, 46≤n≤60, 47≤n≤60, 48≤n≤60, 49≤n≤60, 50≤n≤60, 51≤n≤60, 52≤n≤60, 53≤n≤60, 54≤n≤60, 55≤n≤60, 56≤n≤60, 57≤n≤60, 58≤n≤60, 59≤n≤60, 1≤n≤10, 10≤n≤20, 20≤n≤30, 30≤n≤40, 40≤n≤50, 50≤n≤60, 5≤n≤15, 15≤n≤25, 25≤n≤35, 35≤n≤45, 45≤n≤55, 55≤n≤60, 1≤n≤10, 10≤n≤20, 20≤n≤30, 30≤n≤40, 40≤n≤50, 50≤n≤60, 5≤n≤15, 15≤n≤25, 25≤n≤35, 35≤n≤45, 45≤n≤55, 55≤n≤60, 10≤n≤30, 30≤n≤60, 5≤n≤30, 30≤n≤40, 40≤n≤50, 50≤n≤60, 5≤n≤15, 15≤n≤25, 25≤n≤35, 35≤n≤45, 45≤n≤55, 55≤n≤60, 1≤n≤55, 5≤n≤55, 10≤n≤60, 15≤n≤60, 25≤n≤60, 35≤n≤60, 1≤n≤4, 2≤n≤4, 3≤n≤4, 1≤n≤8, 2≤n≤8, 3≤n≤8, 4≤n≤8, 5≤n≤8, 6≤n≤8, 7≤n≤8, 1≤n≤16, 2≤n≤16, 3≤n≤16, 4≤n≤16, 5≤n≤16, 6≤n≤16, 7≤n≤16, 8≤n≤16, 9≤n≤16, 10≤n≤16, 11≤n≤16, 12≤n≤16, 13≤n≤16, 14≤n≤16, 15≤n≤16, 1≤n≤20, 2≤n≤20, 3≤n≤20, 4≤n≤20, 5≤n≤20, 6≤n≤20, 7≤n≤20, 8≤n≤20, 9≤n≤20, 10≤n≤20, 11≤n≤20, 12≤n≤20, 13≤n≤20, 14≤n≤20, 15≤n≤20, 16≤n≤20, 17≤n≤20, 18≤n≤20, 19≤n≤20, 1≤n≤30, 2≤n≤30, 3≤n≤30, 4≤n≤30, 5≤n≤30, 6≤n≤30, 7≤n≤30, 8≤n≤30, 9≤n≤30, 10≤n≤30, 11≤n≤30, 12≤n≤30, 13≤n≤30, 14≤n≤30, 15≤n≤30, 16≤n≤30, 17≤n≤30, 18≤n≤30, 19≤n≤30, 20≤n≤30, 21≤n≤30, 22≤n≤30, 23≤n≤30, 24≤n≤30, 25≤n≤30, 26≤n≤30, 27≤n≤30, 28≤n≤30, 29≤n≤30, 1≤n≤35, 2≤n≤35, 3≤n≤35, 4≤n≤35, 5≤n≤35, 6≤n≤35, 7≤n≤35, 8≤n≤35, 9≤n≤35, 10≤n≤35, 11≤n≤35, 12≤n≤35, 13≤n≤35, 14≤n≤35, 15≤n≤35, 16≤n≤35, 17≤n≤35, 18≤n≤35, 19≤n≤35, 20≤n≤35, 21≤n≤35, 22≤n≤35, 23≤n≤35, 24≤n≤35, 25≤n≤35, 26≤n≤35, 27≤n≤35, 28≤n≤35, 29≤n≤35, 4≤n≤40, 4≤n≤24, 4≤n≤8, 4≤n≤16, 4≤n≤24, 8≤n≤16, 8≤n≤24, 8≤n≤40, 16≤n≤24, 16≤n≤40, 24≤n≤40, 30≤n≤35, 30≤n≤40, 35≤n≤40 and any range therebetween.

Further, the value of n, or the upper limit value of n, or the lower limit value of n may be 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 and any range therebetween.

In an embodiment, the repeating unit comprises the amino acid sequence as shown in SEQ ID NO.2, and the repetition number n is in the range of 2≤n≤50.

In an embodiment, the repeating unit comprises the amino acid sequence as shown in SEQ ID NO.2, and the repetition number n is in the range of 3≤n≤45.

In an embodiment, the repeating unit comprises the amino acid sequence as shown in SEQ ID NO.2, and the repetition number n is in the range of 4≤n≤40.

In an embodiment, the amino acid residue sequences as shown in SEQ ID NO.1 and SEQ ID NO.2 are derived from a human type XVII collagen, and located at positions 609-638 and 695-724 of the amino acid sequence of the human type XVII collagen, respectively.

Optionally, the collagen may comprise repeating units of the sequences as shown in SEQ ID NO.1 and SEQ ID NO.2 or sequences having 80-100% (for example 80%, 81%, 82%, 83%, 84%, 85%, 86%, 87%, 88%, 89%, 90%, 91%, 92%, 93%, 94%, 95%, 96%, 97%, 98%, 99% or 100%) sequence identity thereto. Each repeating unit may be directly linked. The collagen is a recombinant type XVII collagen having a triple helix structure. The collagen as described herein may have the triple helix structure or three identical chains (i.e., a trimer form). The sequence of each chain may be the sequence as described herein.

Optionally, the amino acid sequence of the collagen comprises an amino acid sequence as shown in SEQ ID NO.3, or an amino acid sequence having at least 90% (for example 90%, 91%, 92%, 93%, 94%, 95%, 96%, 97%, 98%, 99% or 100%) identity to the amino acid sequence as shown in SEQ ID NO.3.

Optionally, the amino acid sequence of the collagen comprises an amino acid sequence as shown in SEQ ID NO.4 or an amino acid sequence having at least 90% (for example 90%, 91%, 92%, 93%, 94%, 95%, 96%, 97%, 98%, 99% or 100%) identity to the amino acid sequence as shown in SEQ ID NO.4.

Optionally, the amino acid sequence of the collagen comprises an amino acid sequence as shown in SEQ ID NO.7 or an amino acid sequence having at least 90% (for example 90%, 91%, 92%, 93%, 94%, 95%, 96%, 97%, 98%, 99% or 100%) identity to the amino acid sequence as shown in SEQ ID NO.7.

Optionally, the amino acid sequence of the collagen comprises an amino acid sequence as shown in SEQ ID NO.8 or an amino acid sequence having at least 90% (for example 90%, 91%, 92%, 93%, 94%, 95%, 96%, 97%, 98%, 99% or 100%) identity to the amino acid sequence as shown in SEQ ID NO.8.

In another aspect, the present invention provides a biological material, comprising any one of the following:
A1) a nucleic acid molecule encoding the recombinant type XVII collagen;
A2) an expression cassette containing the nucleic acid molecule in A1);
A3) a recombinant vector containing the nucleic acid molecule in A1) or the expression cassette in A2); and
A4) a recombinant microorganism containing the nucleic acid molecule in A1), the expression cassette in A2) or the recombinant vector in A3).

Optionally, the expression cassette refers to DNA capable of expressing the aforementioned proteins in a host cell. The expression cassette can also comprise a single-stranded or double-stranded nucleic acid molecule comprising all regulatory sequences necessary for nucleic acid molecules expressing any one of the aforementioned proteins. The regulatory sequences can guide encoding sequences to express any one of the aforementioned proteins in a proper host cell under their compatible conditions. The regulatory sequences include, but are not limited to, a leader sequence, a polyadenylation sequence, a propeptide sequence, a promoter, a signal sequence and a transcription terminator. At a minimum, the regulatory sequences comprise a promoter, as well as transcription and translation termination signals. To introduce specific restriction enzyme sites of a vector so as to link a regulatory sequence to a coding region of a protein-encoding nucleic acid sequence, a regulatory sequence with a linker may be provided. The regulatory sequence may be a proper promoter sequence, i.e., a nucleic acid sequence recognized by a host cell expressing the nucleic acid sequence. The promoter sequence contains a transcriptional regulation sequence mediating protein expression. The promoter may be any nucleic acid sequence having transcriptional activity in the host cell, including mutated, truncated and hybrid promoters, and can be derived from genes encoding extracellular or intracellular proteins homologous or heterologous to the host cell. The regulatory sequence may also be a proper transcription termination sequence, i.e., a sequence that can be recognized by the host cell to terminate transcription. The termination sequence is operatively linked to the 3' end of the nucleic acid sequence encoding the protein. Any terminators functioning in the selected host cell can be used in the present invention. The regulatory sequence may also be a proper leader sequence, i.e., an untranslated region of mRNA that is critical for translation in the host cell. The leader sequence can be operatively linked to the 5' end of the nucleic acid sequence encoding the protein. Any leader sequences functioning in the selected host cell can be used in the present invention. The regulatory sequence may also be a signal peptide coding region that encodes an amino acid sequence linked to the amino terminus of the protein, which directs the encoded protein into the cellular secretory pathway. The signal peptide encoding region capable of directing the expressed protein to enter the secretory pathway of the host cell can be used in the present invention. It may be necessary to add the regulatory sequence for regulating the expression of the protein based on the growth of the host cell. Examples of the regulatory system are those systems that can make response to chemical or physical stimuli (including in the presence of a regulatory compound), thereby activating or repressing gene expression. Other examples of the regulatory sequence are those regulatory sequences allowing the amplification of genes. In these examples, the nucleic acid sequences encoding proteins should be operatively linked to the regulatory sequences.

Optionally, the vector can comprise a nucleic acid molecule encoding the aforementioned protein, a promoter, and transcription and translation termination signals. When the recombinant vector is prepared, the nucleic acid molecule encoding the aforementioned protein is located in the vector so as to be operatively linked to the proper expression regulatory sequence. The recombinant vector can be any vector (for example, a plasmid or a virus) that facilitates recombinant DNA operation and nucleic acid sequence expression. The selection of the vector generally depends on the compatibility of the vector with the host cell into which it is to be introduced. The vector can be a linear or closed circular plasmid. The vector can be an autonomously replicating vector (i.e., the complete structure of the extrachromosomal entity capable of replication independent of chromosomes), for example, a plasmid, an extrachromosomal element, a minichromosome or an artificial chromosome. The vector can comprise any mechanism ensuring self replication. Alternatively, the vector is a vector capable of integrating into the genome and replicating together with the chromosome into which it has integrated upon introduction into a host cell. In addition, a single vector or plasmid, or two or more vectors or plasmids, or transposons, which collectively comprise all DNA to be introduced into the genome of the host cell can be used. The vector comprises one or more selectable markers facilitating the selection of transformed cells. The selectable marker is a gene whose product confers resistance to biocides or viruses, resistance to heavy metals, or prototrophy to an auxotroph, among other properties. Examples of bacterial selectable markers include the dal gene of Bacillus subtilis or Bacillus licheniformis, or resistance markers to antibiotics such as ampicillin, kanamycin, chloramphenicol, or tetracycline. The vector comprises an element capable of integrating into the genome of the host cell, or ensuring that the vector is subjected to autonomous replication in cells independently of a cell genome. As to autonomous replication, the vector may also comprise an origin of replication that enables the vector to autonomously replicate in a target host cell. The origin of replication may carry mutations rendering it temperature-sensitive in the host cell (see, e.g., F.Ehrlich, 1978, Proceedings of the National Academy of Sciences, 75:1433). More than one copy of the nucleic acid molecule of the present invention encoding any of the aforementioned proteins may be inserted into the host cell to increase the yield of the gene product. An increase in the copy number of the nucleic acid molecule can be achieved by inserting at least one additional copy of the nucleic acid molecule into the host cell genome, or by inserting an amplifiable selectable marker together with the nucleic acid molecule, and culturing the cells in the presence of a suitable selection agent to select cells containing an amplified copy of the selectable marker gene and thereby an additional copy of the nucleic acid molecule. The operation for ligating the various elements described above to construct the recombinant expression vectors of the present invention is well known to those skilled in the art (see, e.g., Sambrook et al., Molecular Cloning: A Laboratory Manual, Second Edition, Cold Spring Harbor Laboratory Press, Cold Spring Harbor, NY, 1989).

The term "operatively linked" used herein is defined as such a conformation in which regulatory sequences are located at an appropriate position relative to the coding sequence of a DNA sequence such that the regulatory sequences direct the expression of the protein.

Optionally, the microorganism comprises a polynucleotide of the present invention operatively linked to one or more regulatory sequences that direct the production of the collagen of the present invention. A construct comprising the polynucleotide is introduced into the microorganism, such that the construct is maintained as a chromosomal integrant or as an autonomously replicating extrachromosomal vector. The term "microorganism" encompasses any parent cell progeny that differs from a parent cell due to mutations occurring during replication. The selection of the microorganism largely depends on a gene encoding the collagen or polypeptide and a source thereof.

Optionally, the microorganism is selected from any one of Corynebacterium glutamicum, Bacillus subtilis, Escherichia coli and yeast.

In another aspect, the present invention provides a method for obtaining the recombinant type XVII collagen, comprising the step of expressing the recombinant type XVII collagen using the biological material.

Optionally, the obtaining method can be achieved by microorganism fermentation using the biological material. For example, the nucleic acid molecule encoding the recombinant type XVII collagen is introduced into an empty vector and then transformed into a host cell, and the host cell is incubated for expression.

Optionally, the function of the recombinant type XVII collagen is selected from one or more of the following:
I) improving cell adhesion;
II) regulating the steady state of skin;
III) delaying skin aging;
IV) promoting wound healing; and
V) promoting hair follicle repair.

Optionally, the recombinant type XVII collagen has an electron microscopic structure which is specifically a fibrous structure observed under an electron microscope.

In another aspect, the present invention provides a composition, the composition comprising the recombinant type XVII collagen or the biological material.

Optionally, the composition may further comprise substances of the same type or with similar functions as the recombinant type XVII collagen, and other conventional adjuvants may also be added as needed.

Optionally, the function of the composition is selected from one or more of the following:
I) improving cell adhesion;
II) regulating the steady state of skin;
III) delaying skin aging;
IV) promoting wound healing; and
V) promoting hair follicle repair.

In another aspect, the present invention provides use of the recombinant type XVII collagen, or the recombinant type XVII collagen obtained by using the method, or the composition in the preparation of products for enhancing cell adhesion, and/or regulating skin homeostasis, and/or delaying skin senescence, and/or promoting wound healing, and/or firming, and/or anti-wrinkle, and/or soothing and repairing.

In another aspect, the present invention provides use of the recombinant type XVII collagen or the recombinant type XVII collagen obtained by using the method or the composition in the preparation of products for promoting hair follicle repair and growth, and/or hair care, and/or hair regrowth.

In another aspect, the present invention provides use of the recombinant type XVII collagen or the recombinant type XVII collagen obtained by using the method or the composition in the preparation of hydrating mesotherapy products and/or dermal filler products.

In another aspect, the present invention provides use of the recombinant type XVII collagen or the biological material or the composition in daily chemical products and/or medical device products. The functions of the daily chemical products and/or medical device products are selected from one or more of the following:
I) improving cell adhesion;
II) regulating the steady state of skin;
III) delaying skin aging;
IV) promoting wound healing; and
V) promoting hair follicle repair.

In an embodiment, the recombinant type XVII collagen is used for supporting intracellular or extracellular spaces of animal cells.

In an embodiment, the recombinant type XVII collagen can be used at no higher than 60°C, optionally no higher than 50°C, more optionally no higher than 40°C.

In another aspect, the present invention provides a daily chemical product, comprising the recombinant type XVII collagen or the biological material.

Optionally, the daily chemical product includes, but is not limited to, cosmetics, cleaning products and hair care products.

In another aspect, the present invention provides a medical device product, comprising the recombinant type XVII collagen or the biological material.

Optionally, the medical device product includes, but is not limited to, medical aesthetic products, such as cosmetic injections and lyophilized powder.

Compared with the prior art, the present invention at least has the following benefits:
1. In the present invention, a recombinant type XVII collagen having a triple helix structure capable of self-assembling is successfully screened and obtained by designing and constructing a series of recombinant type XVII collagens. The recombinant type XVII collagen features a simple production process without hydroxylation; and it can be produced by microbial fermentation, exhibits a high expression level and is easy to scale up.
2. The sequence of the recombinant type XVII collagen provided in the present invention is completely derived from human protein sequences. Such recombinant type XVII collagen has low immunogenicity, high safety, stable triple helix structure and strong skin cell supporting capacity. In the efficacy assay, it demonstrates superior abilities in improving cell adhesion, promoting wound healing and promoting hair follicle repair compared with currently available commercial type XVII collagen products, and can be widely used in the fields of cosmetics, medical devices and the like.

### BRIEF DESCRIPTION OF THE DRAWINGS

The accompanying drawings here are incorporated into the specification and constitute a part of the specification, illustrate embodiments consistent with the present invention, and are used for explaining the principles of the present invention together with the specification.

To more clearly illustrate the technical solutions in the embodiments of the present invention or in the prior art, the accompanying drawings used in the embodiments or the prior art description will be briefly introduced below. For those of ordinary skill in the art, other drawings may be obtained from these drawings without creative effort. In the figures:
FIG. 1 is a graph showing CD measurement results of a recombinant type XVII collagen in Example 3 and a commercially available animal collagen, wherein a-e are detection curves at 4°C, 25°C, 37°C, 60°C and 90°C, respectively.
FIG. 2 is a graph showing CD measurement results of a recombinant type XVII collagen in Example 4 at 4°C, 25°C and 37°C, respectively, wherein a is a detection result of XVII-2-1, and b is a detection result of XVII-2-2.
FIG. 3 is a transmission electron microscope image of a recombinant collagen XVII-2 in Example 2.

### DETAILED DESCRIPTION OF THE EMBODIMENTS

To more clearly illustrate the entire concept of the present invention, in the following description, numerous specific details are set forth in order to provide a thorough understanding of the present invention. However, for those skilled in the art, obviously, the present invention is achieved without one or more of these details. In other examples, to avoid confusion with the present invention, certain technical features well known in the art have not been described.

Unless otherwise specified, in the following embodiments, reagents or instruments without indicating manufacturers are all conventional products commercially available. Where no specific conditions are indicated in the examples, experiments are carried out according to conventional conditions or those recommended by the manufacturer.

The plasmids, endonucleases, PCR enzymes, column-based DNA extraction kits, DNA gel recovery kits, and other reagents used in the following examples are all commercial products, and the specific operations are performed according to the respective kit instructions. Unless otherwise stated, the experimental methods, detection methods, and preparation methods disclosed in the present invention all employ conventional techniques of molecular biology, biochemistry, chromatin structure and analysis, analytical chemistry, cell culture, recombinant DNA technology, and conventional techniques in the relevant fields, which may be carried out in accordance with Molecular Cloning: A Laboratory Manual (Fourth Edition).

### Example 1: Design of gene sequence

Two recombinant type XVII collagens, designated as XVII-1 and XVII-2 respectively, were designed by selecting the amino acid residue sequences at positions 609-638 and 695-724 from human type XVII collagen (source UniPort: Q9UMD9) as basic repeating units, and the repetition number was n. See Table 1.

**Table 1 Sequence selection location and repetition number**

| Recombinant protein | Repeating unit | Amino acid number | Repetition number n | Amino acid sequence | Nucleic acid Sequence |
|---|---|---|---|---|---|
| XVII-1 | GSVGDPGMEGPMGQRGREGPM GPRGEΛGPP (SEQ ID NO.1) | 30 | 16 | SEQ ID NO.3 | SEQ ID NO.5 |
| XVII-2 | GVKGDKGPMGPPGPKGDQGEK GPRGLTGEP (SEQ ID NO.2) | 30 | 16 | SEQ ID NO.4 | SEQ ID NO.6 |

### Example 2: Obtainment of recombinant type XVII collagen

### 1. Construction of genetically engineered strains

The nucleic acid molecules of the two recombinant proteins XVII-1 and XVII-2 designed in Table 1 of Example 1 were linked to the BamH1 and Xho1 sites of the vector pET32a respectively to obtain recombinant plasmids pET32a-XVII-1 and pET32a-XVII-2, and then the recombinant plasmids were transferred to an E. coli BL21(DE3) host respectively to obtain genetically engineered strains T-XVII-1 and T-XVII-2. See Table 2.

**Table 2 Strain design and target protein size**

| Strain | Plasmid | Theoretical target protein size (kDa) |
|---|---|---|
| T-XVII-1 | pET32a-XVII-1 | 48 |
| T-XVII-2 | pET32a-XVII-2 | 48 |

### 2. Expression of recombinant type XVII collagen

The single colonies of the genetically engineered strains were picked from the plate and inoculated in 5 mL of LB culture medium (containing 100 µg/mL Amp) for incubation overnight at 37°C and 220 rpm. The above culture broth was transferred to 20 mL of LB culture medium (containing 100 µg/mL Amp) in a ratio of 1:100 and incubated at 37°C and 220 rpm. When the OD₆₀₀ reached 0.6-0.8, 0.2 mM isopropyl β-D-thiogalactopyranoside (IPTG) was added, and then the engineered strains were further incubated at 16°C and 220 rpm for 20 h. Cells corresponding to 10 OD units were collected and suspended in 1 mL of buffer (100 mM Tris-HCl, pH 7.0). The cells were disrupted by ultrasonication. 20 µL of supernatant was taken and mixed with 20 µL of 2× loading buffer. The above mixed solution was boiled for 10 min, and then centrifuged. The resulting supernatant was subjected to sodium dodecyl sulfate-polyacrylamide gel electrophoresis (SDS-PAGE). Meanwhile, quantitative analysis was performed in parallel using bovine serum albumin (BSA). The results are shown in Table 3.

**Table 3 Strain protein expression**

| Strain | Expression level |
|---|---|
| T-XVII-1 | 0.05 g/L |
| T-XVII-2 | 0.19 g/L |

It can be seen from the results in Table 3, both the designed two recombinant proteins XVII-1 and XVII-2 can be expressed in the E. coli host, wherein XVII-2 exhibits the highest expression level, reaching 0.19 g/L.

### 3. Purification of recombinant type XVII collagen

The single colonies of the engineered strains T-XVII-1 and T-XVII-2 were picked from the plate respectively and inoculated into 5 mL of LB culture medium (containing 100 µg/mL Amp) for incubation overnight at 37°C and 220 rpm. The above culture broth was transferred to 100 mL of LB culture medium (containing 100 µg/mL Amp) in a ratio of 1:100 and incubated at 37°C and 220 rpm. When the OD₆₀₀ reached 0.6-0.8, 0.2 mM isopropyl β-D-thiogalactopyranoside (IPTG) was added, and then the engineered strains were further incubated at 16°C and 220 rpm for 20 h. Cells corresponding to 10 OD units were collected and suspended in 1 mL of buffer (100 mM Tris-HCl, pH 7.0). The cells were disrupted by ultrasonication, and centrifuged at 12000 rpm for 60 min to remove pellets. The supernatant was taken for enzyme protein purification. The supernatant was loaded onto a Ni²⁺ agarose affinity chromatography column. The column was washed with 3-4 column volumes of a solution containing Binding buffer (50 mmol/L Tris-HCl, 100 mmol/L NaCl, 25 mmol/L imidazole, pH 7.0), and finally eluted with a solution containing Elution buffer (50 mmol/L Tris-HCl, 400 mmol/L NaCl, 250 mmol/L imidazole, pH 7.0) to obtain a pure enzyme protein. The pure enzyme protein was digested with a tobacco etch virus (TEV) protease overnight at 4°C, and then subjected to Ni column flow through to obtain a purified protein without a label.

### Example 3: Structural characterization of recombinant type XVII collagen

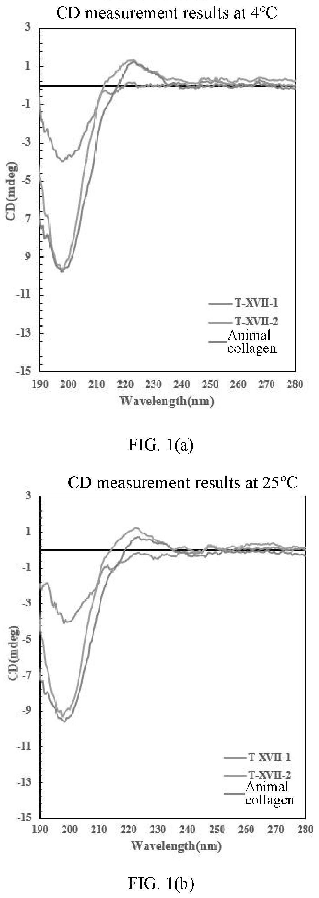

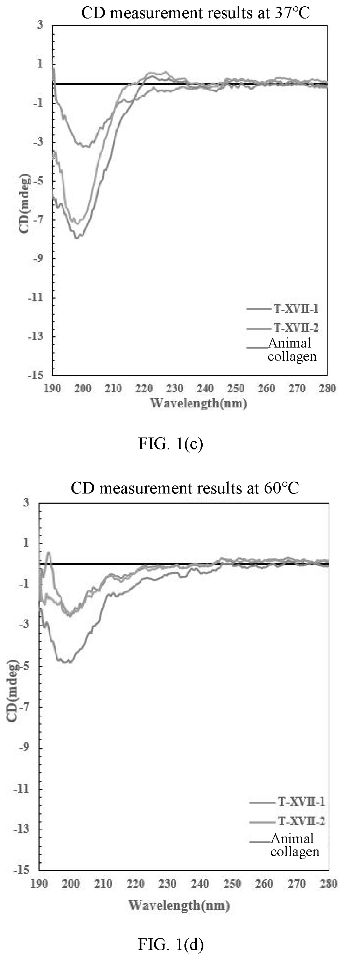

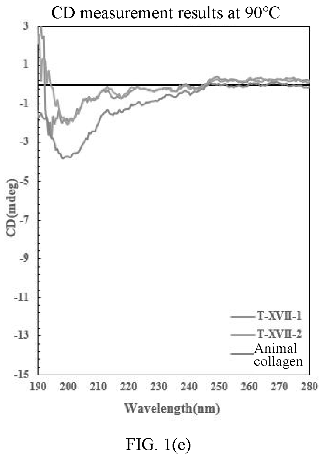

Circular dichroism (CD) measurement was performed using samples derived from T-XVII-1, T-XVII-2 and a commercially available animal collagen sample. The detection conditions were set as follows: a wavelength range of 180 nm-400 nm; the sample was treated in 5 mM PB buffer (pH 7.0) without NaCl. Liquid-phase CD measurement was conducted at different temperature gradients: 4°C, 25°C, 37°C, 60°C and 90°C. The obtained results are shown in FIG. 1

When a negative peak at 195 nm and a positive peak at 221 nm appear in the detection curve, it indicates the presence of the triple helix structure. It can be seen from the results in FIG. 1 that at 4°C, the recombinant proteins XVII-1 and XVII-2 exhibit a triple helix structure identical to that of animal collagen; and at 25°C and 37°C, only XVII-2 still has the same triple helix structure as animal collagen.

### Example 4: Structural characterization of type XVII collagen via electron microscopy

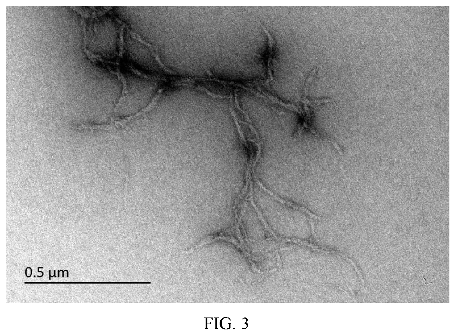

The collagen prepared in Example 2 was detected by transmission electron microscopy; and TEM images were measured and analyzed using ImageJ. The results are shown in FIG. 3.

It can be seen from FIG. 3 that the recombinant type XVII-2 collagen prepared in Example 2 can form a hierarchical fibrous structure.

### Example 5: Structural characterization of recombinant type XVII collagen with varying repetition numbers

By using the recombinant protein XVII-2 as an optional example, multiple recombinant proteins were designed and constructed with repeating units at varying repetition numbers, as shown in Table 4.

**Table 4 Repeating unit and repetition number**

| Recombinant protein | Repeating unit | Amino acid number | Repetition number | Amino acid sequence | Nucleic acid Sequence |
|---|---|---|---|---|---|
| XVII-2-1 | GVKGDKGPMGPP GPKGDQGEKGPR GLTGEP | 30 | 4 | SEQ ID NO.7 | SEQ ID NO.9 |
| XVII-2-2 | GVKGDKGPMGPP GPKGDQGEKGPR GLTGEP | 30 | 8 | SEQ ID NO.8 | SEQ ID NO.10 |

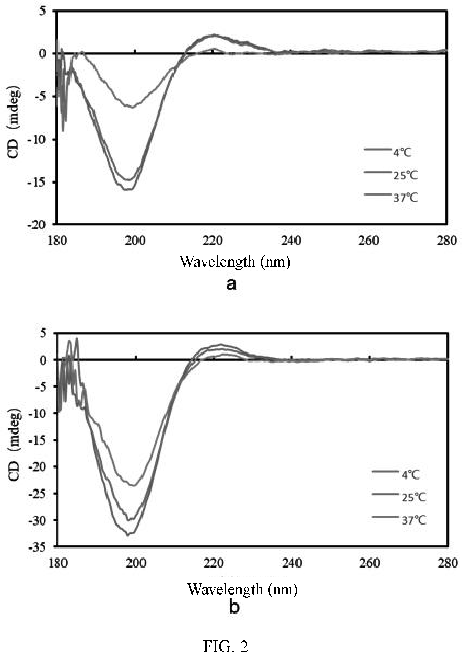

According to the design of the recombinant proteins in Table 4, the recombinant proteins were prepared and purified by using the method in Example 2, and the structural characterization of the recombinant proteins was carried out by using the detection method in Example 3. The obtained results are shown in FIG. 2.

It can be seen from the results in FIG. 2 that the recombinant proteins XVII-2-1 and XVII-2-2 have a triple helix structure.

### Example 6: Functional assay

### 1. Cell adhesion detection

Cell adhesion capability detection was performed on the collagens prepared in Example 2 and Example 5. The steps are as follows:

Cells were incubated to the logarithmic growth phase, digested with 0.25% (m/v) trypsin, and suspended in a serum-free DMEM culture medium. The cell concentration was adjusted to 5×10⁵ cells/mL, and then the cells were inoculated into a 24-well plate coated with five collagens, respectively. 500 µL of cell suspension was added to each well, resulting in 2.5×10⁵ cells per well. Each group was set up in triplicate, followed by incubation in a cell incubator (37°C, 5% CO₂) for 24 h. After 24 h, the incubation was terminated. The culture medium was discarded, and the cells were washed with PBS 2-3 times, followed by discarding PBS. 1 mL of paraformaldehyde (PFA) was added to each well, the cells were fixed for 15 min, and then the fixative was discarded. 1 mL of 0.1% crystal violet solution was added to each well for 20 min of staining. The staining solution was gently rinsed away with running water, and cell adhesion was photographed under a microscope. Cell staining areas were analyzed using ImageJ, and the average value of each replicate well was calculated. The calculation formula of the cell adhesion rate is as follows:
$Cell adhesion rate = \left(Cell crystal violet staining area/Total area\right) \times 100 %$

The results are shown in Table 5.

**Table 5 Cell adhesion rate**

| | Cell adhesion rate (%) |
|---|---|
| Control | 104.85% ± 4.78% |
| XVII-1 | 139.39% ± 9.99% |
| XVII-2 | 162.45% ± 12.07% |
| XVII-2-1 | 160.05% ± 9.08% |
| XVII-2-2 | 157.20% ± 11.02% |
| Commercially available recombinant collagen | 41.79% ± 7.23% |

It can be seen from Table 5 that the recombinant proteins prepared in Example 2 and Example 5 have a cell adhesion capability that is significantly superior to that of the commercially available recombinant collagen.

### 2. Cell scratch assay

Cell scratch assay was performed to evaluate the scratch repair ability of the collagens prepared in Example 2 and Example 5. The steps are as follows:
Cells were incubated to the logarithmic growth phase, digested with 0.25% (m/v) trypsin, and suspended in a serum-free DMEM culture medium. The cell concentration was adjusted to 5×10⁵ cells/mL, and 1 mL of cell suspension was inoculated to each well of the 24-well plate, resulting in 5×10⁵ cells per well. Each group was set up in triplicate. Once the cells were incubated in a complete culture medium to 80% confluence, a scratch was made to cells in each well. Human fibroblasts (HSF) were treated with five collagens, and the cell confluence at the scratch was imaged using an inverted microscope at 0 h, 12 h and 24 h, respectively. The cell area in the scratch region was analyzed using ImageJ, and the proportion of the cell migration area was calculated. The values from each replicate well were averaged. The calculation formula of the proportion of the cell migration area is as follows:
$Proportion of cell migration area = \left(Cell confluent area/Scratch area\right) \times 100 %$

The obtained results are shown in Table 6.

**Table 6 Cell migration rate**

| Migration time | Class | Cell migration rate (%) |
|---|---|---|
| 12-hour migration rate | Control | 31.10% ± 3.48% |
| | XVII-1 | 55.20% ± 3.70% |
| | XVII-2 | 41.20% ± 3.40% |
| | XVII-2-1 | 45.21% ± 4.04% |
| | XVII-2-2 | 41.18% ± 5.12% |
| | Commercially available recombinant collagen | 37.33% ± 2.52% |
| 24-hour migration rate | Control | 57.67% ± 2.52% |
| | XVII-1 | 78.93% ± 2.57% |
| | XVII-2 | 68.10% ± 2.00% |
| | XVII-2-1 | 70.20% ± 3.22% |
| | XVII-2-2 | 71.35% ± 4.21% |
| | Commercially available recombinant collagen | 65.67% ± 3.06% |

It can be seen from the results in Table 6 that compared with the commercially available recombinant collagen and control group, the recombinant collagens provided in the present invention exhibit higher cell migration rates and are suitable for the preparation of related functional products.

### 3. Detection on the regulation of functional genes related to hair follicle repair

Hair follicle-related gene detection was performed on the recombinant collagen prepared in Example 2 using a qPCR technology. The steps are as follows:
Cells were incubated to the logarithmic growth phase, digested with 0.25% (m/v) trypsin, and suspended in a serum-free DMEM culture medium. The cell concentration was adjusted to 5×10⁵ cells/mL, and then the cells were inoculated in a 6-well plate. 2 mL of cell suspension was added into each well, resulting in 1×10⁶ cells per well. Each group was performed in triplicate. Once the cells were incubated to 80% confluence in a complete culture medium, human dermal papilla cells were treated with a 1 mg/mL collagen sample for 24 h (37°C, 5% CO₂). Cell RNA was then extracted for qPCR. Total RNA was isolated from cell pellets using a TRizol reagent, and RNA was reverse-transcribed into cDNA using a cDNA reverse transcription kit. The mRNA expression levels of WNT10a, β-catenin and VEGFA were determined and analyzed on a real-time quantitative PCR system using a reaction mixture prepared from SybrGreen mix and primers. Relative mRNA expression was analyzed using GAPDH as an internal reference.

The obtained results are shown in Table 7.

**Table 7 Functional genes related to hair follicle repair**

| Group | Relative Wnt10a mRNA expression | Relative β-catenin mRNA expression | Relative VEGFA mRNA expression |
|---|---|---|---|
| Control | 1.04 ± 0.11 | 1.02 ± 0.04 | 1.06 ± 0.09 |
| XVII-1 | 2.94 ± 0.41 | 1.58 ± 0.12 | 2.46 ± 0.19 |
| XVII-2 | 2.56 ± 0.33 | 1.87 ± 0.22 | 2.58 ± 0.28 |
| Commercially available recombinant collagen | 1.54 ± 0.15 | 1.87 ± 0.21 | 1.15 ± 0.04 |

It can be seen from the results in Table 7 that compared with the commercially available recombinant collagen, both the recombinant proteins XVII-1 and XVII-2 can activate the hair follicle repair-related gene Wnt10a, stimulate hair follicle stem cells, and induce dormant hair follicles to enter the anagen phase. Furthermore, XVII-1 and XVII-2 can also activate the β-catenin gene to promote hair follicle repair. Meanwhile, XVII-1 and XVII-2 can also upregulate the expression of vascular endothelial growth factor (VEGFA), induce angiogenesis in the dermal papilla to regulate the hair follicle cycle, and maintain and promote hair follicle growth. Therefore, the recombinant proteins XVII-1 and XVII-2 exhibit excellent ability to promote hair follicle repair and growth, and are suitable for the preparation of related functional products.

### 4. Detection of recombinant type XVII collagen in promoting the expression of other collagens

By taking XVII-2 collagen as an example, the effects of the XVII-2 collagen on the levels of Collagen I, Collagen III and MMP-9 were detected. The steps are as follows:
When the cell seeding efficiency in a 24-well plate reached 40-60%, human fibroblasts were subjected to UVA irradiation. The negative control group, positive control group and sample group were subjected to UVA irradiation at 9 J/cm², while the blank control group was placed under the same conditions (with a UVA irradiation dose of 0 J/cm²). After irradiation, 1 mL of cell culture medium was added to each well for the blank control (BC) group and the negative control (NC) group. 1 mL of culture medium containing 100 µg/mL VC and 7 µg/mL VE was added to each well for the positive control (PC) group. 1 mL of XVII-2 recombinant protein sample at various concentrations was added to each well for the sample group. Following incubation for 24 h, cell culture supernatant was collected and placed in an EP tube, and then stored frozen at -80°C. The levels of Collagen I, Collagen III and MMP-9 were detected and analyzed according to the manufacturer's instruction of the ELISA kit. The obtained results are shown in Table 8.

**Table 8 Effects of XVII-2 on other collagens and MMP-9**

| Group | Type I collagen level (ng/mL) | Type III collagen level (ng/mL) | MMP-9 level (ng/mL) |
|---|---|---|---|
| BC | 115.57 ± 4.45 | 34.47 ± 1.21 | 1.32 ± 0.04 |
| NC | 71.69 ± 0.82 | 19.17 ± 0.38 | 2.58 ± 0.06 |
| PC | 111.48 ± 9.92 | 35.85 ± 1.59 | 1.22 ± 0.05 |
| 0.05% XVII-2 | 120.83 ± 9.87 | 37.97 ± 1.08 | 1.64 ± 0.03 |
| 0.1% XVII-2 | 144.33 ± 1.09 | 51.31 ± 3.93 | 1.49 ± 0.00 |

It can be seen from the results in Table 8 that the recombinant collagen XVII-2 at different concentrations can significantly increase the levels of Collagen I in human fibroblasts, promote the expression of type III collagen, and markedly reduce the level of matrix metalloproteinase MMP-9 in human fibroblasts. That is to say, the XVII-2 collagen can promote the expression of type I collagen and type III collagen and significantly reduce the expression of MMP-9, indicating that the XVII-2 protein possesses anti-wrinkle and skin-firming effects.

The above descriptions are merely embodiments of the present invention, but are not intended to limit the present invention. For those skilled in the art, various alternations and variations can be made to the present invention. Any modifications, equivalent replacements, improvements and the like made within the spirit and principle of the present invention shall fall within the scope of the appended claims of the present invention.

## Claims

1. A recombinant type XVII collagen, wherein the collagen has an amino acid sequence containing one or more amino acid residue repeating units, wherein the repeating unit comprises a sequence as shown in SEQ ID NO. 1 or SEQ ID NO.2, and the repetition number is n, where 1<n≤60.

2. The recombinant type XVII collagen according to claim 1, wherein the repeating unit comprises an amino acid sequence as shown in SEQ ID NO. 2, and the repetition number n is in the range of 2≤n≤18.

3. The recombinant type XVII collagen according to claim 1 or 2, wherein the amino acid sequence of the collagen comprises an amino acid sequence as shown in SEQ ID NO. 3 or an amino acid sequence having at least 90% identity to the amino acid sequence as shown in SEQ ID NO. 3, and/or an amino acid sequence as shown in SEQ ID NO. 4 or an amino acid sequence having at least 90% identity to the amino acid sequence as shown in SEQ ID NO. 4, and/or an amino acid sequence as shown in SEQ ID NO. 7 or an amino acid sequence having at least 90% identity to the amino acid sequence as shown in SEQ ID NO. 7, and/or an amino acid sequence as shown in SEQ ID NO. 8 or an amino acid sequence having at least 90% identity to the amino acid sequence as shown in SEQ ID NO. 8.

4. A biological material, wherein the biological material is selected from any one of the following:
A1) a nucleic acid molecule encoding the recombinant type XVII collagen according to any one of claims 1-3;
A2) an expression cassette containing the nucleic acid molecule in A1);
A3) a recombinant vector containing the nucleic acid molecule in A1) or the expression cassette in A2); and
A4) a recombinant microorganism containing the nucleic acid molecule in A1), the expression cassette in A2) or the recombinant vector in A3).

5. A method for obtaining a recombinant type XVII collagen, comprising the step of expressing the recombinant type XVII collagen using the biological material according to claim 4.

6. A composition, the composition comprising the recombinant type XVII collagen according to any one of claims 1-3 or the recombinant type XVII collagen obtained by using the method according to claim 5.

7. Use of the recombinant type XVII collagen according to any one of claims 1-3 or the recombinant type XVII collagen obtained by using the method according to claim 5 or the composition according to claim 6 in the preparation of daily chemical products, medical device products, pharmaceutical products and/or tissue engineering products.

8. Use of the recombinant type XVII collagen according to any one of claims 1-3 or the recombinant type XVII collagen obtained by using the method according to claim 5 or the composition according to claim 6 in the preparation of products for enhancing cell adhesion, and/or regulating skin homeostasis, and/or delaying skin senescence, and/or promoting wound healing, and/or firming, and/or anti-wrinkle, and/or soothing and repairing.

9. Use of the recombinant type XVII collagen according to any one of claims 1-3 or the recombinant type XVII collagen obtained by using the method according to claim 5 or the composition according to claim 6 in the preparation of products for promoting hair follicle repair and growth, and/or hair care, and/or hair regrowth.

10. Use of the recombinant type XVII collagen according to any one of claims 1-3 or the recombinant type XVII collagen obtained by using the method according to claim 5 or the composition according to claim 6 in the preparation of hydrating mesotherapy products and/or dermal filler products.
